(19) Europäisches Patentamt
European Patent Office
Office européen des brevets

(11) Veröffentlichungsnummer: **0 162 246**
**B1**

(12) # EUROPÄISCHE PATENTSCHRIFT

(45) Veröffentlichungstag der Patentschrift: **25.11.87**

(21) Anmeldenummer: **85103980.0**

(22) Anmeldetag: **02.04.85**

(51) Int. Cl.⁴: **C 07 D 249/08**, C 07 D 233/60, A 01 N 47/38 // C07C87/28, C07C125/03

(54) **N-(Arylpropyl)-azolylharnstoffe und diese enthaltende Fungizide.**

(30) Priorität: **21.04.84 DE 3415139**

(43) Veröffentlichungstag der Anmeldung: **27.11.85 Patentblatt 85/48**

(45) Bekanntmachung des Hinweises auf die Patenterteilung: **25.11.87 Patentblatt 87/48**

(84) Benannte Vertragsstaaten: **AT BE CH DE FR GB IT LI NL SE**

(56) Entgegenhaltungen:
**DE-A-1 136 342**
**GB-A-1 469 772**
**GB-A-2 011 414**
**US-A-4 046 773**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Rentzea, Costin, Dr., Richard-Kuhn-Strasse 1-3, D-6900 Heidelberg (DE)**
Erfinder: **Buschmann, Ernst, Dr., Georg-Ludwig-Krebs-Strasse 10, D-6700 Ludwigshafen (DE)**
Erfinder: **Himmele, Walter, Dr., Eichenweg 14, D-6909 Walldorf (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

**EP 0 162 246 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue N-(Arylpropyl)-azolylharnstoffe, Verfahren zu ihrer Herstellung und fungizide Mittel, die diese Verbindungen als Wirkstoff enthalten.

Es ist bekannt, N-Trichlormethylthio-tetrahydrophthalimid und N-Trichlormethylthiophthalimid als Fungizide in der Landwirtschaft sowie im Obst- und Gartenbau einzusetzen (Chemical Week, 1972, June 21, Seite 63 und Seite 46). Die bekannten Mittel sind jedoch nur vor der Infektion verwendbar und ihre Wirkungen genügen bei niedrigen Aufwandmengen den Anforderungen der Praxis nicht.

Es ist ferner bekannt, di-N-substituierte Carbamoyl-triazole und di-N-substituierte Carbamoyl-imidazole als Fungizide zu verwenden (GB-A-1 469 772 und GB-A-2 011 414). Die fungizide Wirkung der Verbindungen, die den Phenylethylrest als Substituenten aus Carbamoylstickstoff enthalten, ist aber nur gering.

Es wurde nun gefunden, daß N-A(Arylpropyl)-azolylharnstoffe der Formel

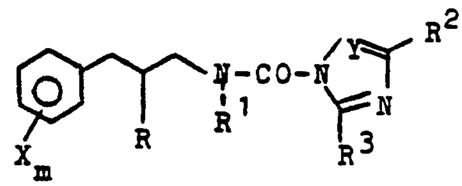 (I),

in der

R Alkyl mit 1 bis 4 C-Atomen bedeutet,

X Wasserstoff, Halogen, Nitro, Cyan, Trifluormethyl oder Alkyl, Alkoxy und Alkylthio jeweils 1 bis 6 C-Atomen oder Phenyl oder Phenoxy bedeutet,

m eine ganze Zahl von 1 bis 4 bedeutet, wobei die einzelnen Gruppen X gleich oder verschieden sind, wenn m größer als 1 ist,

Y CH oder N bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$-$C_4$ Alkyl, $C_1$-$C_4$ Alkoxy, Trifluormethyl, Nitro oder Cyan substituierten Phenyl- oder Benzylrest bedeutet, und

$R^2$ und $R^3$ gleich oder verschieden sind und Wasserstoff oder Alkyl mit 1 bis 5 C-Atomen bedeuten,

sehr gut wirksam gegen Schadpilze sind.

Die neuen Verbindungen der Formel I enthalten chirale Zentren und werden im allgemeinen in Form von Racematen oder als Diastereomerengemische erhalten. Die Diastereomeren lassen sich bei einigen der neuen Verbindungen beispielsweise durch Säulenchromatographie trennen oder aufgrund von Löslichkeitsunterschieden in reiner Form isolieren. Aus solchen einheitlichen Diastereomeren kann man mit bekannten Methoden einheitliche Enantiomere erhalten. Die Gemische und die einheitlichen Verbindungen werden von der vorliegenden Erfindung umfaßt. Für die Anwendung der neuen Verbindungen als Fungizide sind sowohl die einheitlichen Diastereomeren bzw. Enantiomeren wie auch deren bei der Synthese anfallenden Gemische geeignet, bevorzugt werden die letzteren verwendet.

X bedeutet vorzugsweise Wasserstoff, Fluor, Chlor, Brom, Cyan, Nitro, Trifluormethyl, Methyl, Ethyl, n-Propyl, Isopropyl, n-Butyl, Isobutyl, sek.-Butyl, tert.-Butyl, Methoxy, Ethoxy, Methylthio, Phenyl oder Phenoxy.

Als Bedeutung für $R^1$ in Formel I kommen Reste wie Methyl, Ethyl, n-Propyl, n-Butyl, Isobutyl, sek.-Butyl, n-Pentyl, 3-Methyl-1-butyl, 2-Methyl-1-butyl, 3-Pentyl, n-Hexyl, 3,3-Dimethyl-1-butyl, 2,2,3-Trimethyl-1-propyl, n-Heptyl, n-Octyl, 2,4,4-Trimethylpentyl, 2-Ethylhexyl, n-Nonyl, 3,5,5-Trimethylhexyl, 2-Isopropyl-5-methylhexyl, 2-Isopropyl-3-methylhexyl, n-Decyl, 3,7-Dimethyloctyl, Dodecyl, Cyclopropylmethyl, Cyclopentyl, Cyclohexyl, Cyclohexylmethyl, 4-Methylcyclohexyl, 4-tert.-Butylcyclohexyl, 4-Methoxycyclohexyl, 2-Methoxyethyl, 2-Ethoxyethyl, 2-Propoxyethyl, 2-Butoxyethyl, 2-Hexyloxyethyl, 3-Methoxypropyl, 3-Ethoxypropyl, 3-Propoxypropyl, 3-Butoxypropyl, 3-Hexyloxypropyl, 2-Methylthioethyl, 2-Ethylthioethyl, 2-Propylthioethyl, 2-Butylthioethyl, 3-Methylthiopropyl, 3-Ethylthiopropyl, 3-Propylthiopropyl, Allyl, 2-Methylallyl, 2-Buten-1-yl, 2-Penten-1-yl, Phenyl, 4-Fluorphenyl, 4-Chlorphenyl, 2,4-Dichlorphenyl, 3,4-Dichlorphenyl, 4-Bromphenyl, 4-Methylphenyl, 4-tert.-Butylphenyl, 4-Methoxyphenyl, 4-Ethoxyphenyl, 4-Trifluormethyl, 4-Cyanphenyl, Benzyl, 4-Fluorbenzyl, 4-Chlorbenzyl, 3,4-Dichlorbenzyl, 2,4-Dichlorbenzyl, 4-Brombenzyl, 4-Methylbenzyl, 4-tert.-Butylbenzyl, 4-Methoxybenzyl, 3- und 4-Trifluormethylbenzyl, Phenylethyl und 4-Ethoxybenzyl, in Frage.

$R^2$ und $R^3$ bedeuten vorzugsweise Wasserstoff, Methyl, Ethyl, n-Propyl oder Isopropyl und

R Methyl und Ethyl,

Y bedeutet Stickstoff oder CH und

m die ganze Zahl 1, 2, 3 oder 4.

Die Verbindungen der Formel I lassen sich herstellen, indem man ein Carbamoylchlorid der Formel

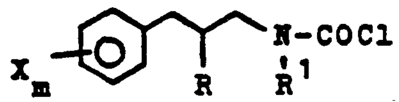

(II),

in der R, $R^1$ und X die oben angegebemen Bedeutungen haben,
a) mit Azolen der Formel III

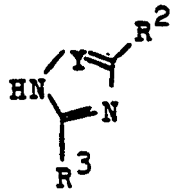

(III),

in der $R^2$, $R^3$ und Y die oben angegebenen Bedeutungen haben, oder
b) mit deren Metall-Derivaten der Formel IV

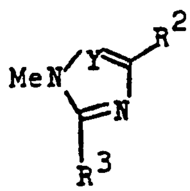

(IV),

in der $R^2$, $R^3$ und Y die oben angegebenen Bedeutungen haben und
Me Lithium, Natrium, Kalium oder 1/2 Calcium bedeutet, oder
c) mit deren Silyl-Derivaten der Formel V

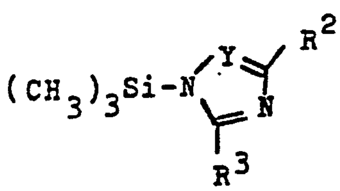

(V),

in der $R^2$, $R^3$ und Y die oben angegebenen Bedeutungen haben, umsetzt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120° C.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium-, Natrium-, Kalium- oder Calciumhydroxid, Alkalicarbonate wie Natrium- oder Kaliumcarbonat oder Natrium- und Kaliumhydrogencarbonat, Amine wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktiomsbeschleuniger kommen vorzugsweise Metallhalogenide wie Natriumiodid oder Kaliumiodid, quarternäre Ammoniumsalze wie Tetrabutylammonium-chlorid, -bromid oder -iodid, Benzyltriethylammomiumchlorid oder -bromid oder Kronenether wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei

einer Temperatur im Bereich zwischen 0 und 140°C, vorzugsweise zwischen 0 und 100°C. Hierfur eignen sich dieselben Lösumgsmittel wie für die Verfahrensvariante a).

Die Verbindungen der Formel I lassen sich ferner herstellen, inden man ein sekundäres Amin der Formel VI

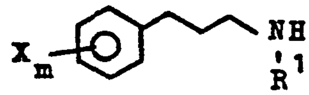 (VI),

in der R, $R^1$ und X die oben angegebenen Bedeutungen haben, mit eimem Carbonyl-bisazol der Formel VII

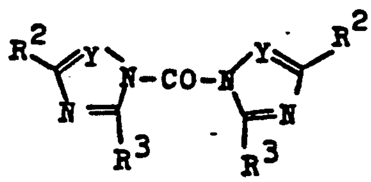 (VII),

in der $R^2$, $R^3$ und Y die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünungsmittel: Diethylether, 1,2-Dimethoxyethan, Dipropylether, Dibutylether, Methyl-tert.-butylether, Tetrahydrofuran, Dimethoxyethan, Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylethylketon.

Geeignete Reaktionsbeschleuniger sind beispielsweise 4-Dimethylaminopyridin oder 4-Pyrrolidinopyridin.

Die Ausgangsstoffe der Formel II können leicht nach bekannten Verfahren hergestellt werden, beispielsweise durch Umsetzung von Aminen der Formel VI mit Phosgen (Houben-Weyl-Müller, Methoden der organischen Chemie, Band 8, Seite 115 bis 118, Georg Thieme Verlag, Stuttgart, 1952).

Die sekundären Amine der Formel VI werden durch Umsetzung von bekannten Aminen der Formel $R^1$-$NH_2$, in der $R^1$ die oben angegebene Bedeutung hat, mit Arylpropylhalogeniden (s. EP 9077) der Formel VIII oder mit Aldehyden der Formel IX erhalten

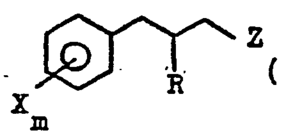 (VIII) 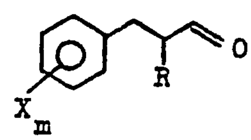 (IX),

in der R, X und m die oben angegebenen Bedeutungen haben und Z Chlor oder Brom bedeutet, gegebenenfalls in Gegenwart einer starken anorganischen oder organischen Base, gegebenenfalls in Gegenwart eines Lösungs- oder Verdünnungsmittels und gegebenenfalls in Gegenwart von hydrierenden Verbindungen und/oder Katalysatoren.

Die folgenden Beispiele erläutern die Herstellung der Verbindungen der Formel I:

**Beispiel I**

a) Einer Lösung von 71 g n-Butylamin (1 Mol) in 800 ml Ethanol werden 204 g 3-(4'-tert.-Butylphenyl)-2-methylpropionaldehyd bei 25°C zugetropft. Nach 36 stündigem Rühren bei 25°C wird das Gemisch portionsweise mit 87 g (2,28 Mol) Natriumborhydrid versetzt und weitere 4 Stunden bei 78°C gerührt. Das Gemisch wird abgekühlt, mit 1,5 l Wasser verdünnt und dreimal mit je 300 ml Methylenchlorid extrahiert. Die vereinigten Extrakte werden mit 300 ml Wasser gewaschen, getrocknet und destilliert.

Man erhält 170 g N-Butyl-N-3-(1-(4'-tert.-butylphenyl)-2-methyl)-propylamin vom Siedepunkt 125°C/0,4 mbar.

b) Zu einer bei 10°C gesättigten Lösung von Phosgen in 200 ml trockenem Essigester werden 51,2 g (0,196 Mol) N-Butyl-N-3-(1-(4'-tert.-butylphenyl)-2-methyl)-propylamin zugetropft. Das Gemisch wird bei 50°C 6 Stunden unter Phosgeneinleitung gerührt, anschließend auf 20°C abgekühlt und im Vakuum - schließlich bei 60°C und 0,4 mbar - eingeengt.

Man erhält 61 g N-3-(1-(4'-tert.-Butylphenyl)-2-methyl)-propyl-N-butylcarbamoylchlorid als farbloses Öl, das gleich weiter umgesetzt wird.

c) Zu einer Lösung von 32,4 g (0,1 Mol) N-3-(1-(4'-tert.-Butylphenyl)-2-methyl)-propyl-N-butylcarbamoylchlorid in 250 ml trockenem Tetrahydrofuran werden portionsweise 20,4 g (0,3 Mol) Imidazol zugegeben. Nach 6-stündigem Rühren bei 70°C wird das Gemisch auf 20°C abgekühlt und der gebildete

Niederschlag abgesaugt. Das Filtrat wird unter vermindertem Druck eingeengt, der Rückstand in 200 ml Methylenchlorid aufgenommen, dreimal mit je 80 ml Wasser gewaschen, getrocknet und eingeengt.

Man erhält 29,2 g 1-(N-3-(1-(4'-tert.-Butylphenyl)-2-methyl)-propyl-N-butylcarbamoyl)-imidazol als farbloses Harz (Verbindung Nr. 1). IR (Film) [$cm^{-1}$]: 2961, 2933, 2872, 1694, 1464, 1421, 1365, 1286, 1272, 1231, 1100, 1066, 1020, 750, 658.

**Beispiel 2**

Zu einer Suspension von 5,5 g (0,06 Mol) Natrium-1,2,4-triazolid in 120 ml trockenem Tetrahydrofuran werden bei 20°C 14 g (0,0433 Mol) N-3-(1-(4'-tert.-Butylphenyl)-2-methyl)-propyl-N-butylcarbamoylchlorid zugetropft. Nach 6-stündigem Rühren bei 65°C wird das Gemisch auf 20°C abgekühlt und der Niederschlag abgesaugt. Das Filtrat wird eingeengt und der Rückstand in 150 ml Methylenchlorid aufgelöst, dreimal mit je 50 ml Wasser gewaschen, getrocknet und eingeengt. Man erhält 13,3 g 1-(N-3-(1-(4'-tert.-Butylphenyl)-2-methyl)-propyl-N-butylcarbamoyl)-1,2,4-triazol als farbloses Harz (Verbindung Nr. 2). IR (Film) [$cm^{-1}$]: 2961, 2931, 2871, 1699, 1508, 1464, 1426, 1379, 1364, 1275, 1210, 1192, 1136, 1001, 671.

In entsprechender Weise können die in der folgenden Tabelle aufgeführten Verbindungen hergestellt werden:

| Verbindung Nr. | X | R | $R^1$ | Y | $R^2$ | $R^3$ | Fp[°C] IR (Film) [$cm^{-1}$] |
|---|---|---|---|---|---|---|---|
| 3 | H | $CH_3$ | n-$C_4H_9$ | CH | H | H | |
| 4 | H | $CH_3$ | n-$C_4H_9$ | N | H | H | |
| 5 | H | $C_2H_5$ | n-$C_4H_9$ | CH | H | H | |
| 6 | H | $C_2H_5$ | n-$C_4H_9$ | N | H | H | |
| 7 | H | $CH_3$ | n-$C_6H_{13}$ | CH | H | H | |
| 8 | H | $CH_3$ | n-$C_6H_{13}$ | N | H | H | |
| 9 | H | $CH_3$ | Cyclopropylmethyl | CH | H | H | |
| 10 | H | $CH_3$ | Cyclopropylmethyl | N | H | H | |
| 11 | H | $CH_3$ | $-(CH_2)_2OC_2H_5$ | CH | H | H | |
| 12 | H | $CH_3$ | $-(CH_2)_2OC_2H_5$ | N | H | H | |
| 13 | 4-F | $CH_3$ | n-$C_3H_7$ | CH | H | H | 2963, 1692, 1509, 1462, 1421, 1298, 1276, 1242, 1221, 1099, 1013, 760, 656, |
| 14 | 4-F | $CH_3$ | n-$C_3H_7$ | N | H | H | 2966, 1699, 1510, 1427, 1220, 1001, 671 |
| 15 | 4-F | $CH_3$ | $-(CH_2)_3OC_4H_5$-n | CH | H | H | |
| 16 | 4-F | $CH_3$ | $-(CH_2)_3O-C_4H_5$-n | N | H | H | |
| 17 | 4-F | $CH_3$ | $-C_6H_5$ | CH | H | H | |
| 18 | 4-F | $CH_3$ | $-C_6H_5$ | N | H | H | |
| 19 | 4-Cl | $CH_3$ | n-$C_6H_{13}$ | CH | H | H | $n^{22}_D$ 1,5342 |
| 20 | 4-Cl | $CH_3$ | n-$C_6H_{13}$ | N | H | H | $n^{22}_D$ 1,5279 |
| 21 | 4-Cl | $CH_3$ | $-(CH_2)_2CH(CH_3)CH_2C(CH_3)_3$ | CH | H | H | |
| 22 | 4-Cl | $CH_3$ | $-(CH_2)_2CH(CH_3)CH_2C(CH_3)_3$ | N | H | H | |
| 23 | 4-Br | $CH_3$ | n-$C_4H_9$ | CH | H | H | |
| 24 | 4-Br | $CH_3$ | n-$C_4H_9$ | N | H | H | |
| 25 | 4-$CH_3O$- | $CH_3$ | n-$C_3H_7$ | CH | H | H | 2962, 2933, 1692, 1611, 1512, 1462, 1421, 1299, 1275, 1221, 1178, 1034, 754 |
| 26 | 4-$CH_3O$- | $CH_3$ | n-$C_3H_7$ | N | H | H | 2963, 1698, 1512, 1426, 1276, 1247, 1179, 1001 |
| 27 | 4-$C_2H_5O$- | $CH_3$ | n-$C_6H_{13}$ | CH | H | H | |
| 28 | 4-$C_2H_5O$- | $CH_3$ | n-$C_6H_{13}$ | N | H | H | |
| 29 | 4-$C_6H_{13}O$- | $CH_3$ | n-$C_4H_9$ | CH | H | H | |
| 30 | 4-$C_6H_{13}O$- | $CH_3$ | n-$C_4H_9$ | N | H | H | |
| 31 | 4-$CH_3$ | $CH_3$ | n-$C_3H_7$ | CH | H | H | 2963, 2930, 1693, |

| | | | | | | | |
|---|---|---|---|---|---|---|---|
| | | | | | | | 1515, 1461, 1421, 1380, 1364, 1298, 1275, 1220, 1100, 1015, 754 |
| 32 | 4-$CH_3$ | $CH_3$ | n-$C_3H_7$ | N | H | H | 2965, 1699, 1428, 1381, 1275, 1138, 1001, 748 |
| 33 | 4-$C_2H_5$ | $CH_3$ | n-$C_6H_{13}$ | CH | H | H | |
| 34 | 4-$C_2H_5$ | $CH_3$ | n-$C_6H_{13}$ | N | H | H | |
| 35 | 4-iso$C_3H_7$ | $CH_3$ | n-$C_4H_9$ | CH | H | H | |
| 36 | 4-iso$C_3H_7$ | $CH_3$ | n-$C_4H_9$ | N | H | H | |
| 37 | 4-iso$C_3H_7$ | $CH_3$ | n-$C_6H_{13}$ | CH | H | H | |
| 38 | 4-iso$C_3H_7$ | $CH_3$ | n-$C_6H_{13}$ | N | H | H | |
| 39 | 4-tert.-$C_4H_9$ | $CH_3$ | $CH_3$ | CH | H | H | 70 - 72 |
| 40 | 4-tert.-$C_4H_9$ | $CH_3$ | $CH_3$ | CH | H | iso$C_3H_7$ | 2965, 2931, 2870, 1701, 1510, 1476, 1461, 1364, 1244, 1231,1108 |
| 41 | 4-tert.-$C_4H_9$ | $CH_3$ | $CH_3$ | N | H | H | 2961, 2869, 1704, 1508, 1478, 1406, 1380, 1364, 1275, 1197, 1131, 998, 746, 671 |
| 42 | 4-tert.-$C_4H_9$ | $CH_3$ | $C_2H_5$ | CH | H | H | 2963, 1695, 1461, 1422, 1328, 1249, 1220, 1018, 900, 758 |
| 43 | 4-tert.-$C_4H_9$ | $CH_3$ | $C_2H_5$ | N | H | H | |
| 44 | 4-tert.-$C_4H_9$ | $CH_3$ | n-$C_3H_7$ | CH | H | H | 2963, 2873, 1693, 1462, 1420, 1365, 1272, 1242, 1100, 1065, 1017, 755, 658 |
| 45 | 4-tert.-$C_4H_9$ | $CH_3$ | n-$C_3H_7$ | CH | H | iso$C_3H_7$ | 2964, 2872, 1693, 1511, 1460, 1417, 1380, 1363, 1346, 1269, 1109, 1096, 1070 |
| 46 | 4-tert.-$C_4H_9$ | $CH_3$ | n-$C_3H_7$ | N | H | H | 2963, 2873, 1700, 1509, 1462, 1426, 1380, 1364, 1274, 1244, 1211, 1001, 671 |
| 47 | 4-tert.-$C_4H_9$ | $CH_3$ | n-$C_4H_9$ | CH | H | iso$C_3H_7$ | 2962, 2872, 1695, 1460, 1418, 1379, 1363, 1288, 1270, 1109, 1096, 1070, 718 |
| 48 | 4-tert.-$C_4H_9$ | $CH_3$ | $-(CH_2)_2CH(CH_3)_2$ | CH | H | H | |
| 49 | 4-tert.-$C_4H_9$ | $CH_3$ | $-(CH_2)_2CH(CH_3)_2$ | N | H | H | |
| 50 | 4-tert -$C_4H_9$ | $CH_3$ | n-$C_6H_{13}$ | CH | H | H | $n_D^{23}$ 1,5205 |
| 51 | 4-tert.-$C_4H_9$ | $CH_3$ | n-$C_6H_{13}$ | N | H | H | |
| 52 | 4-tert.-$C_4H_9$ | $CH_3$ | $-CH_2CH(C_2H_5)(CH_2)_3CH_3$ | CH | H | H | |
| 53 | 4-tert.-$C_4H_9$ | $CH_3$ | $-CH_2CH(C_2H_5)(CH_2)_3CH_3$ | N | H | H | |
| 54 | 4-tert.-$C_4H_9$ | $CH_3$ | n-$C_7H_{15}$ | CH | H | H | |
| 55 | 4-tert.-$C_7H_{15}$ | $CH_3$ | n-$C_7H_{15}$ | N | H | H | |
| 56 | 4-tert.-$C_4H_9$ | $CH_3$ | 4-Cl-$C_6H_4$- | CH | H | H | 1701, 1494 1416, 1394, 1287, 1251, 1095, 1015, 846, 750, 735, 654, 570 |
| 57 | 4-tert.-$C_4H_9$ | $CH_3$ | 4-Cl-$C_6H_4$- | CH | H | iso$C_3H_7$ | 1701, 1493, 1414, 1393, 1289, 1269, 1095, 1070, 1016, 838, 759, 738, |

| | | | | | | | |
|---|---|---|---|---|---|---|---|
| | | | | | | | 723, 608 |
| 58 | 4-tert.-$C_4H_9$ | $CH_3$ | 4-Cl-$C_6H_4$- | N | H | H | 1708, 1509 1493, 1458, 1440, 1420, 1400, 1380, 1281, 1092, 1014, 990 |
| 59 | 4-tert.-$C_4H_9$ | $CH_3$ | Cyclohexyl | CH | H | H | |
| 60 | 4-tert.-$C_4H_9$ | $CH_3$ | Cyclohexyl | N | H | H | 2933, 1700, 1508, 1421, 1273, 1003, 731, 671 |
| 61 | 4-tert.-$C_4H_9$ | $CH_3$ | 4-Methylcyclohexyl | CH | H | H | 2858, 1695, 1410, 1240, 1100, 1019, 732, 657 |
| 62 | 4-tert.-$C_4H_9$ | $CH_3$ | 4-Methylcyclohexyl | N | H | H | |
| 63 | 4-$C(CH_3)_2C_2H_5$ | $CH_3$ | n-$C_6H_{13}$ | CH | H | H | $n_D^{22}$ 1,5168 |
| 64 | 4-$C(CH_3)_2C_2H_5$ | $CH_3$ | n-$C_6H_{13}$ | N | H | H | $n_D^{22}$ 1,5111 |
| 65 | 4-$CF_3$ | $CH_3$ | n-$C_6H_{13}$ | CH | H | H | |
| 66 | 4-$CF_3$ | $CH_3$ | n-$C_6H_{13}$ | N | H | H | |
| 67 | 4-iso$C_3H_7$ | $CH_3$ | $CH_3$ | CH | H | H | 2959, 2927, 1696, 1512, 1484, 1459, 1402, 1382, 1282, 1217, 1099, 1009, 754, 656 |
| 68 | 4-iso$C_3H_7$ | $CH_3$ | $CH_3$ | N | H | H | 2959, 2927, 1703, 1510, 1479, 1406, 1381, 1363, 1276, 1198, 1131, 998, 671 |
| 69 | 2,4-$Cl_2$ | CH | n-$C_3H_7$ | CH | H | H | 2963, 2933, 1693, 1473, 1420, 1382, 1299, 1275, 1242, 1103, 1066, 1015, 744, 657 |
| 70 | 2,4-$Cl_2$ | $CH_3$ | n-$C_3H_7$ | N | H | H | 2965, 1698, 1474, 1426, 1382, 1276, 1244, 1105, 1001, 746, 670 |
| 71 | 2,4-$Cl_2$ | $CH_3$ | $-CH_2-CH=CH_2$ | CH | H | H | |
| 72 | 2,4-$Cl_2$ | $CH_3$ | $-CH_2-CH=CH_2$ | N | H | H | |
| 73 | 2,4-$Cl_2$ | $CH_3$ | $-CH_2-CH=CH-CH_3$ | CH | H | H | |
| 74 | 2,4-$Cl_2$ | $CH_3$ | $-CH_2-CH=CH-CH_3$ | N | H | H | |
| 75 | 2,4-$Cl_2$ | $CH_3$ | $-CH_2-CH=CH-C_3H_6$ | CH | H | H | |
| 76 | 2,4-$Cl_2$ | $CH_3$ | iso-$C_4H_9$ | CH | H | H | 2960, 1693, 1472, 1419, 1385, 1277, 1244, 1103, 1065, 1018, 745, 657 |
| 77 | 2,4-$Cl_2$ | $CH_3$ | iso-$C_4H_9$ | N | H | H | 2961, 1698, 1473, 1381, 1275, 1003, 670 |
| 78 | 2,4-$Cl_2$ | $CH_3$ | n-$C_{12}H_{25}$ | CH | H | H | 2953, 2924, 2853, 1695, 1472, 1420, 1379, 1299, 1274, 1240, 1220, 1103, 1065, 744 |
| 79 | 2,4-$Cl_2$ | $CH_3$ | n-$C_{12}H_{25}$ | N | H | H | 2924, 1699, 1472, 1426, 1275, 1000, 744, 670 |
| 80 | 2,4-$Cl_2$ | $CH_3$ | Cyclohexyl | CH | H | H | 2933, 1694, 1474, 1409, 1377, 1303, 1240, 1103, 1020, 746 |
| 81 | 2,4-$Cl_2$ | $CH_3$ | Cyclohexyl | N | H | H | 65 - 67 |
| 82 | H | n-$C_3H_7$ | n-$C_6H_{13}$ | CH | H | H | $n_D^{25}$ 1,5321 |
| 83 | H | n-$C_3H_7$ | n-$C_6H_{13}$ | N | H | H | $n_D^{25}$ 1,5136 |
| 84 | H | n-$C_4H_9$ | n-$C_4H_9$ | CH | H | H | $n_D^{22}$ 1,5224 |
| 85 | H | n-$C_4H_9$ | n-$C_4H_9$ | N | H | H | $n_D^{22}$ 1,5172 |

| | | | | | | | |
|---|---|---|---|---|---|---|---|
| 86 | H | n-$C_4H_9$ | n-$C_6H_{13}$ | CH | H | H | $n_D^{20}$ 1,5178 |
| 87 | H | n-$C_4H_9$ | n-$C_6H_{13}$ | N | H | H | $n_D^{20}$ 1,5125 |
| 88 | 2-F | $CH_3$ | n-$C_4H_9$ | CH | H | H | $n_D^{22}$ 1,5262 |
| 89 | 2-F | $CH_3$ | n-$C_4H_9$ | NH | H | H | $n_D^{22}$ 1,5180 |
| 90 | 2-F | $CH_3$ | n-$C_6H_{13}$ | CH | H | H | $n_D^{22}$ 1,5192 |
| 91 | 4-Cl | $CH_3$ | n-$C_4H_9$ | CH | H | H | $n_D^{23}$ 1,5428 |
| 92 | 4-$CH_3$ | $CH_3$ | Cyclohexyl | CH | H | H | 2930, 1694, 1515, 1409, 1376, 1241, 1100, 1020, 757 |
| 93 | 4-$CH_3$ | $CH_3$ | Cyklohexyl | N | H | H | 2931, 1700, 1507, 1422, 1378, 1274, 1178, 1003, 671 |
| 94 | 4-$C(CH_3)_3$ | $CH_3$ | n-$C_4H_9$ | CH | H | H | 2959, 1694, 1420, 1364, 1271, 1230, 1020, 656 |
| 95 | 4-$C(CH_3)_3$ | $CH_3$ | n-$C_4H_9$ | N | H | H | 2960, 1699, 1426, 1379, 1274, 1210, 1001, 671 |
| 96 | 4-$C(CH_3)_3$ | $CH_3$ | n-$C_8H_{17}$ | CH | H | H | $n_D^{25}$ 1,5158 |
| 97 | 4-$C(CH_3)_3$ | $CH_3$ | n-$C_8H_{17}$ | N | H | H | $n_D^{22}$ 1,5096 |
| 98 | 4-$C(CH_3)_3$ | $CH_3$ | n-$C_5H_{11}$ | CH | H | H | $n_D^{23}$ 1,5176 |
| 99 | 4-$C(CH_3)_3$ | $CH_3$ | n-$C_5H_{11}$ | N | H | H | $n_D^{23}$1,5135 |
| 100 | 4-$C(CH_3)_3$ | $CH_3$ | $-HC(CH_3)CH_2CH_2CH_3$ | CH | H | H | $n_D^{23}$ 1,5268 |
| 101 | 4-$C(CH_3)_3$ | $CH_3$ | $-HC(CH_3)CH_2CH_2CH_3$ | N | H | H | $n_D^{23}$ 1,5192 |
| 102 | 4-$C(CH_3)_3$ | $CH_3$ | iso-$C_4H_9$ | CH | H | H | $n_D^{22}$ 1,5190 |
| 103 | 4-$C(CH_3)_3$ | $CH_3$ | iso-$C_4H_9$ | N | H | H | 2960, 1694, 1419, 1267, 1244, 1099, 1018, 804 |
| 104 | 4-$C(CH_3)_3$ | $CH_3$ | $(CH_3)_2CHCH_2CH_2$- | CH | H | H | $n_D^{23}$ 1,5215 |
| 105 | 4-$C(CH_3)_3$ | $CH_3$ | $-HC(CH_3)C_5H_{11}$-n | CH | H | H | 2960, 1694, 1408, 1375, 1325, 1298, 1099, 759 |
| 106 | 4-$C(CH_3)_2C_2H_5$ | $CH_3$ | n-$C_4H_9$ | CH | H | H | $n_D^{20}$1,5242 |
| 107 | 4-$C(CH_3)_2C_2H_5$ | $CH_3$ | n-$C_4H_9$ | N | H | H | $n_D^{22}$ 1,5183 |
| 108 | 4-$C(CH_3)_2C_2H_5$ | $CH_3$ | n-$C_6H_{13}$ | CH | H | H | $n_D^{25}$ 1,5194 |
| 109 | 4-$C(CH_3)_2C_2H_5$ | $CH_3$ | n-$C_6H_{13}$ | N | H | H | $n_D^{25}$ 1,5146 |
| 110 | 4-$C(CH_3)_2C_3H_7$n | $CH_3$ | n-$C_4H_9$ | CH | H | H | $n_D^{25}$ 1,5221 |
| 111 | 4-$C(CH_3)_2C_3H_7$n | $CH_3$ | n-$C_4H_9$ | N | H | H | $n_D^{25}$ 1,5172 |
| 112 | 4-$C(CH_3)_2C_3H_7$n | $CH_3$ | n-$C_6H_{13}$ | CH | H | H | $n_D^{23}$ 1,5154 |
| 113 | 4-$C(CH_3)_2C_3H_7$-i | $CH_3$ | n-$C_4H_9$ | CH | H | H | $n_D^{26}$ 1,5176 |
| 114 | 4-$C(CH_3)_2C_3H_7$-i | $CH_3$ | n-$C_4H_9$ | N | H | H | $n_D^{26}$ 1,5187 |
| 115 | 4-$C(CH_3)_2C_3H_7$-i | $CH_3$ | n-$C_6H_{13}$ | CH | H | H | $n_D^{26}$ 1,5187 |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, wie Coniophora puteana und Polystictus versicolor, verwendet werden. Darüber hinaus können mit den neuen Wirkstoffen auch Schimmelpilze und holzverbläuende Pilze wie Pullularia pullulans effektiv bekämpft wrrden. Außerdem sind die neuen Wirkstoffe auch gegen Candida albicans und Trichophyton mentogrophytes wirksam.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:

Pseudocercosporella herpotrichoides in Getreide
Erysiphe graminis (echter Mehltau) in Getreide,
Erysiphe cichoriacearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,

Septoria nodorum an Weizen,
Botrytis cinerea (Grauschimmel) an Erdbeeren, Reben,
Rhynchosporium secalis und
Pyrenophora teres in Getreide.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin.
1-(Butylcarbamoyl)-2-benzimidazol-carbaminsäuremethylester
2-Methoxycarbonylamino-benzimidazol
2-(Furyl-(2))-benzimidazol
2-(Thiazolyl-(4))-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäureanilid
2-Methyl-furan-3-carbonsäureanilid
2,5-Dimethyl-furan-3-carbonsäureanilid
2,4,5-Trimethyl-furan-3-carbonsäureanilid

2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diyl-bis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harmstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanon
alpha-(2-Chlorphenyl-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)1,2-dimethylcyclopropan-1,2-dicarbonsäureimid
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z, B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalisulfonsäuren, Phenolsulfönsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes

Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung 25 werden in einer Mischung gelöst, die aus 80 Gewichtsteilen Xylol, 10 Gewichtsteilen des Anlagerungs-Produktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der nach Beispiel 39 erhältlichen Verbindungen werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 44 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 80 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 40 erhältlichen Verbindung werden mit 95 Gewichtsteilen feinteiligem Kaolin innig vermischt. Man erhält auf diese Weise ein Stäubemittel, das 5 Gew.-% des Wirkstoffs erhält.

VII. 30 Gewichtsteile der Verbindung 76 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 41 werden mit 30 Teilen Natriumsalz eines Phenolsulfonsäure-Harnstoff- Formaldehyd-Kondensates, 2 Teilen Kieselgel und 48 Teilen Wasser innig vermischt. Man erhält eine stabile wäßrige Dispersion.

IX. 20 Teile der Verbindung 46 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel sind die bekannten insbesondere zur Bekämpfung von Botrytis geeigneten Wirkstoffe N-Trichlormethylthio-tetrahydrophthalimid (A) und N-Trichlormethylthiophthalimid (B).

**Versuch 1**

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05 %-ige Wirkstoffbrühe die Verbindungen 1, 25, 39, 44 und 80 eine bessere fungizide Wirkung zeigten (beispielsweise 90 %) als der bekannte Wirkstoff A (beispielsweise 70 %).

**Versuch 2**

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt.

Dann erfolgt die Beurteilung des Ausmaßes des Pilzausbruches auf den Blattunterseiten.

Das Ergebnis des Versuches zeigt, daß beispielsweise bei der Anwendung als 0,05 %-ige Wirkstoffbrühe die Verbindungen 40, 44 und 76 eine bessere fungizide Wirkung zeigten (beispielsweise 97 %) als der bekannte Wirkstoff B (beispielsweise 90 %).

**Versuch 3**

Wirksamkeit gegen Pyricularia oryzae (protektiv)

Blätter von in Töpfen gewachsenen Reiskeimlingen der Sorte "Bahia" werden mit wäßrigen Emulsionen, die 80 % Wirkstoff und 20 % Emulgiermittel in der Trockensubstanz enthalten, tropfnaß besprüht und 24 Stunden später mit einer wäßrigen Sporensuspension von Pyricularia oryzae inoculiert. Anschließend werden die Versuchspflanzen in Klimakammern bei 22 bis 24°C und 95 bis 99 % relativer Luftfeuchtigkeit aufgestellt. Nach 6 Tagen wird das Ausmaß des Krankheitsbefalls ermittelt.

Das Ergebnis des Versuches zeigt, daß beispielsweise bei der Anwendung als 0,05 %-ige Wirkstoffbrühe die Verbindungen 2, 40, 41, 46, 58, 67, 68, 69 und 70 eine gut fungizide Wirkung zeigten (beispielsweise 97 %).

**Patentansprüche** für die Vertragsstaaten BE, CH, DE, FR, GB, IT, LI, NL, SE.

1. N-(Arylpropyl)-azolylharnstoff der Formel

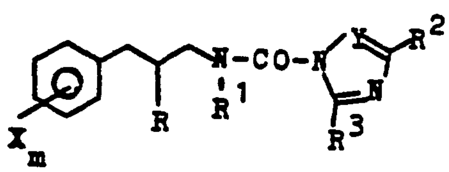 (I),

in der

R Alkyl mit 1 bis 4 C-Atomen bedeutet,

X Wasserstoff, Halogen, Nitro, Cyan, Trifluormethyl oder Alkyl, Alkoxy und Alkylthio jeweils mit 1 bis 6 C-Atomen oder Phenyl oder Phenoxy bedeutet,

m eine ganze Zahl von 1 bis 4 bedeutet, wobei die einzelnen Gruppen X gleich oder verschieden sind, wenn m größer als 1 ist,

Y CH oder N bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und

$R^2$ und $R^3$ gleich oder verschieden sind und Wasserstoff oder Alkyl mit 1 bis 5 C-Atomen bedeuten.

2. Fungizides Mittel, enthaltend einen N-(Arylpropyl)-azolylharnstoff der Formel I gemäß Anspruch 1.

3. Fungizides Mittel, enthaltend einen inerten Zusatzstoff und einen N-(Arylpropyl)-Azolylharnstoff der Formel I gemäß Anspruch 1.

4. Verfahren zur Herstellung eines fungiziden Mittels, dadurch gekennzeichnet, daß man einen inerten Zusatzstoff vermischt mit einem N-(Arylpropyl)-Azolylharnstoff der Formel I gemäß Anspruch 1.

5. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge eines N-(Arylpropyl)-azolylharnstoffs der Formel I gemäß Anspruch 1.

6. Verfahren zur Herstellung von N-(Arylpropyl)-azolylharnstoffen der Formel I gemäß Anspruch 1, dadurch gekennzeichnet, daß man eine Verbindung der Formel

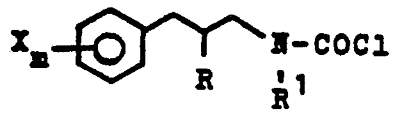 (II),

in der R, $R^1$ und X die im Anspruch 1 angegebenen Bedeutungen haben,

a) mit einem Azol der Formel

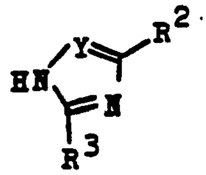 (III),

in der $R^2$, $R^3$ und Y die im Anspruch 1 angegebenen Bedeutungen haben, oder

b) mit dessem Metall-Derivat der Formel

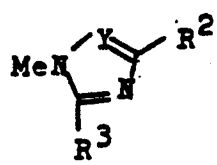 (IV),

in der $R^2$, $R^3$ und Y die im Anspruch 1 angegebenen Bedeutungen haben und

Me Lithium, Natrium, Kalium oder 1/2 Calcium bedeutet, oder

c) mit deren Silyl-Derivaten der Formel

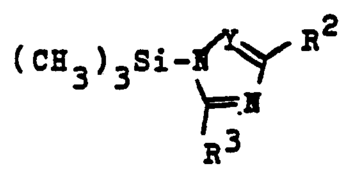 (V),

in der $R^2$, $R^3$ und Y die im Anspruch 1 angegebenen Bedeutungen haben, umsetzt.

7. Verfahren zur Herstellung von N-(Arylpropyl)-azolylharnstoffen der Formel I gemäß Anspruch 1, dadurch gekennzeichnet, daß man eine Verbindung der Formel

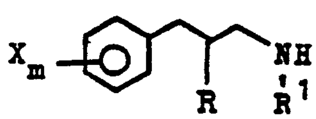 (VI),

in der R, $R^1$ und X die in Anspruch 1 angegebenen Bedeutungen haben, mit einem Carbonyl-bisazol der Formel

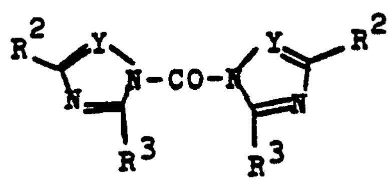 (VII),

in der $R^2$, $R^3$ und Y die im Anspruch 1 angegebenen Bedeutungen haben, umsetzt.

8. N-(Arylpropyl)-azolylharnstoffe der Formel I gemäß Anspruch 1, dadurch gekennzeichnet, daß

X Wasserstoff, Fluor, Chlor, Brom, Cyan, Nitro, Trifluormethyl, Methyl, Ethyl, n-Propyl, Isopropyl, n-Butyl, Isobutyl, sek.-Butyl, tert.-Butyl, Methoxy, Ethoxy, Methylthio, Phenyl, oder Phenoxy bedeutet,

R Methyl und Ethyl,

$R^2$ und $R^3$ Wasserstoff, Methyl, Ethyl, n-Propyl oder Isopropyl bedeuten,

Y CH oder Stickstoff ist, und

$R^1$ Methyl, Ethyl, n-Propyl, n-Butyl, Isobutyl, sek.-Butyl, n-Pentyl, 3-Methyl-1-butyl, 2-Methyl-1-butyl, 3-Pentyl,

n-Hexyl, 3,3-Dimethyl-1-butyl, 2,2,3-Trimethylpropyl, n-Heptyl, n-Octyl, 2,4,4-Trimethylpentyl, 2-Ethylhexyl, n-Nonyl, 3,5,5-Trimethylhexyl, 2-Isopropyl-5-methylhexyl, n-Decyl, 3,7-Dimethyloctyl, Dodecyl, Cyclopropylmethyl, Cyclopentyl, Cyclohexyl, Cyclohexylmethyl, 4-Methylcyclohexyl, 2-Methoxyethyl, 2-Ethoxyethyl, 2-Propoxyethyl, 2-Butoxyethyl, 3-Meth-oxypropyl, 3-Ethoxypropyl, 3-Propoxypropyl, 3-Butoxypropyl, 2-Methylthioethyl, 2-Ethylthioethyl, 3-Methylthiopropyl, Allyl, 2-Buten-1-yl, 2-Penten-1-yl, Phenyl, p-Chlorphenyl, p-Methoxyphenyl, p-Methylphenyl, Benzyl, p-Chlorbenzyl, 2,4-Dichlorbenzyl, p-Methylbenzyl oder Phenylethyl bedeutet und

m eine ganze Zahl von 1 bis 4 ist.

**Patentansprüche** für den Vertragsstaat AT

1. Fungizides Mittel, enthaltend N-(Arylpropyl)-azolylharnstoff der Formel

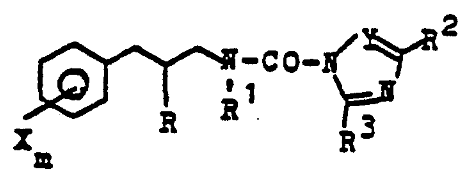 (I),

in der

R Alkyl mit 1 bis 4 C-Atomen bedeutet,

X Wasserstoff, Halogen, Nitro, Cyan, Trifluormethyl oder Alkyl, Alkoxy und Alkylthio jeweils mit 1 bis 6 C-Atomen oder Phenyl oder Phenoxy bedeutet,

m eine ganze Zahl vom 1 bis 4 bedeutet, wobei die einzelnen Gruppen X gleich oder verschieden sind, wenn m größer als 1 ist,

Y CH oder N bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen gegebenenfalls durch Halogen, $C_1$-$C_4$-Alkyl, $C_1$-$C_4$-Alkoxy, Trifluormethyl, Nitro oder Cyan substituirten Phenyl- oder Benzylrest bedeutet, und

$R^2$ und $R^3$ gleich oder verschieden sind und Wasserstoff oder Alkyl mit 1 bis 5 C-Atomen bedeuten.

2. Fungizides Mittel, enthaltend einem inerten Zusatzstoff und einem N-(Arylpropyl)-azolylharnstoff der Formel I gemäß Anspruch 1.

3. Verfahren zur Herstellung eines fungiziden Mittels, dadurch gekennzeichnet, daß man einen inerten Zusatzstoff vermischt mit einem N-(Arylpropyl)-azolylharnstoff der Formel I gemäß Anspruch 1.

4. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge eines N-(Arylpropyl)-azolylharnstoffs der Formel I gemäß Anspruch 1.

5. Verfahren zur Herstellung von N-(Arylpropyl)-azolylharnstoffen der Formel I gemäß Anspruch 1, dadurch gekennzeichnet, daß man eine Verbindung der Formel

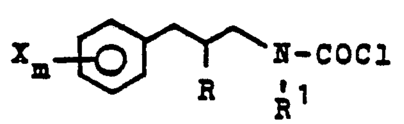 (II),

in der R, $R^1$ und X die im Anspruch 1 angegebenen Bedeutungen haben,

a) mit einem Azol der Formel

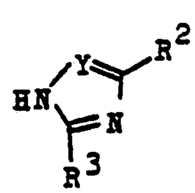 (III),

in der $R^2$, $R^3$ und Y die im Anspruch 1 angegebennn Bedeutungen haben, oder

b) mit dessem Metall-Derivat der Formel

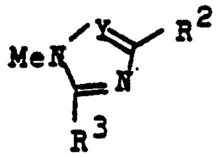 (IV),

in der $R^2$, $R^3$ und Y die im Anspruch 1 angegebenen Bedeutungen haben und Me Lithium, Natrium, Kalium oder 1/2 Calcium bedeutet, oder

c) mit deren Silyl-Derivaten der Formel

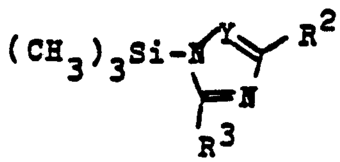 (V),

in der $R^2$, $R^3$ und Y die im Anspruch 1 angegebenen Bedeutungen haben, umsetzt.

6. Verfahren zur Herstellung von N-(Arylpropyl)-azolylharnstoffen der Formel I gemäß Anspruch 1, dadurch gekennzeichnet, daß man eine Verbindung der Formel

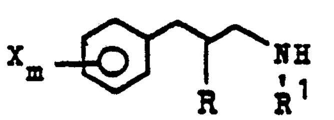 (VI),

in der R, $R^1$ und Y die im Anspruch 1 angegebenen Bedeutungen haben, mit einem Carbonyl-bisazol der Formel

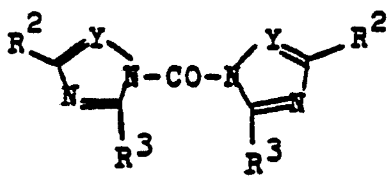 (VII),

in der $R^2$, $R^3$ und Y die im Anspruch 1 angegebenen Bedeutungen haben, umsetzt.

**Claims** for contract states BE, CH, DE, FR, GB, IT, LI, NL, SE

1. An N-(arylpropyl)-azolylurea of the formula

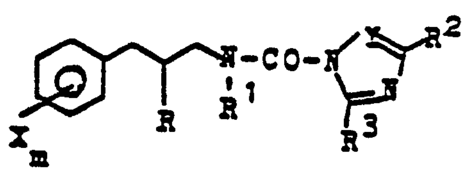 (I)

where

R is alkyl of 1 to 4 carbon atoms,

X is hydrogen, halogen, nitro, cyano, trifluoromethyl or alkyl, alkoxy or alkylthio, each of which is of 1 to 6 carbon atoms, or phenyl or phenoxy,

m is an integer from 1 to 4, the individual groups X being identical or different when m is greater than 1,

Y is CH or N,

$R^1$ is alkyl, alkenyl, alkoxyalkyl, alkylthioalkyl, cycloalkyl or cycloalkylalkyl, each of not more than 12 carbon atoms, or is phenyl or benzyl, each of which is unsubstituted or substituted by halogen, $C_1$-$C_4$-alkyl, $C_1$-$C_4$-alkoxy, trifluoromethyl, nitro or cyano, and

$R^2$ and $R^3$ are identical or different and are each hydrogen or alkyl of 1 to 5 carbon atoms.

2. A fungicidal agent containing an N-(arylpropyl)-azolylurea of the formula I as claimed in claim 1.

3. A fungicidal agent containing an inert additive and an N-(arylpropyl)-azolylurea of the formula I as claimed in claim 1.

4. A process for the preparation of a fungicidal agent, wherein an inert additive is mixed with an N-(arylpropyl)-azolylurea of the formula I as claimed in claim 1.

5. A process for combating fungi, wherein the fungi or the materials, areas, plants or seed threatened by fungal attack are treated with a fungicidally effective amount of an N-(arylpropyl)-azolylurea of the formula I as claimed in claim 1.

6. A process for the preparation of an N-(arylpropyl)-azolylurea of the formula I as claimed in claim 1, wherein a compound of the formula

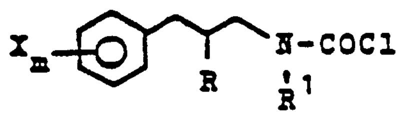 (II),

where R, $R^1$ and X have the meanings given in claim 1, is reacted

a) with an azole of the formula

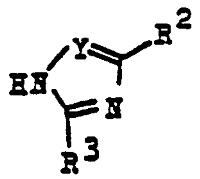 (III),

where $R^2$, $R^3$ and Y have the meanings given in claim 1, or

b) with its metal derivative of the formula

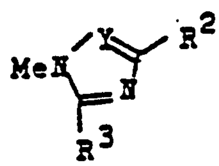 (IV),

where $R^2$, $R^3$ and Y have the meanings given in claim 1 and Me is lithium, sodium, potassium or 1/2 calcium, or

c) with its silyl derivative of the formula

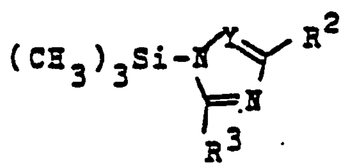 (V),

where $R^2$, $R^3$ and Y have the meanings given in claim 1.

7. A process for the preparation of an N-(arylpropyl)-azolylurea of the formula I as claimed in claim 1, wherein a compound of the formula

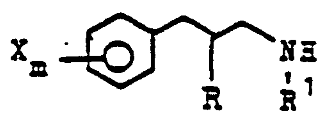 (VI),

where R, $R^1$ and X have the meanings given in claim 1, is reacted with a carbonylbisazole of the formula

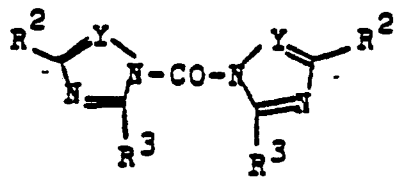 (VII),

where $R^2$, $R^3$ and Y have the meanings given in claim 1.

8. An N-(arylpropyl)-azolylurea of the formula I as claimed in claim 1 wherein

X is hydrogen, fluorine, chlorine, bromine, cyano, nitro, trifluoromethyl, methyl, ethyl, n-propyl, isopropyl, n-butyl, isobutyl, sec-butyl, tert-butyl, methoxy, ethoxy, methylthio, phenyl or phenoxy,

R is methyl or ethyl,

$R^2$ and $R^3$ are hydrogen, methyl, ethyl, n-propyl or isopropyl,

Y is CH or nitrogen, and

$R^1$ is methyl, ethyl, n-propyl, n-butyl, isobutyl, sec-butyl, n-pentyl, 3-methylbut-1-yl, 2-methylbut-1-yl, 3-pentyl, n-hexyl, 3,3-dimethylbut-1-yl, 2,2,3-trimethylpropyl, n-heptyl, n-octyl, 2,4,4-trimethylpentyl, 2-ethylhexyl, n-nonyl, 3,5,5-trimethylhexyl, 2-isopropyl-5-methylhexyl, n-decyl, 3,7-dimethyloctyl, dodecyl, cyclopropylmethyl, cyclopentyl, cyclohexyl, cyclohexylmethyl, 4-methylcyclo-hexyl, 2-methoxyethyl, 2-ethoxyethyl, 2-propoxyethyl, 2-butoxyethyl, 3-methoxypropyl, 3-ethoxypropyl, 3-propoxypropyl, 3-butoxypropyl, 2-methylthioethyl, 2-ethylthioethyl, 3-methylthiopropyl, allyl, but-2-en-1-yl, pent-2-en-1-yl, phenyl, p-chlorophenyl, p-methoxyphenyl, p-methylphenyl, benzyl, p-chlorobenzyl, 2,4-dichlorobenzyl, p-methylbenzyl or phenylethyl, and

m is an integer from 1 to 4.

**Claims** for contract state AT

1. A fungicidal agent containing an N-(arylpropyl)-azolylurea of the formula

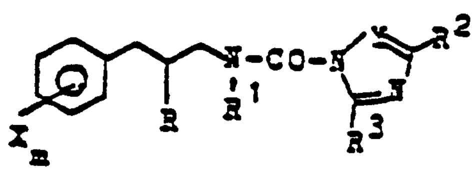 (I),

where

R is alkyl of 1 to 4 carbon atoms,

X is hydrogen, halogen, nitro, cyano, trifluoromethyl or alkyl, alkoxy or alkylthio, each of which is of 1 to 6 carbon atoms, or phenyl or phenoxy,

m is an integer from 1 to 4, the individual groups X being identical or different when m is greater than 1,

Y is CH or N,

$R^1$ is alkyl, alkenyl, alkoxyalkyl, alkylthioalkyl, cycloalkyl or cycloalkylalkyl, each of not more than 12 carbon atoms, or is phenyl or benzyl, each of which is unsubstituted or substituted by halogen, $C_1$-$C_4$-alkyl, $C_1$-$C_4$-alkoxy, trifluoromethyl, nitro or cyano, and

$R^2$ and $R^3$ are identical or different and are each hydrogen or alkyl of 1 to 5 carbon atoms.

2. A fungicidal agent containing an inert additive and an N-(arylpropyl)-azolylurea of the formula I as claimed in claim 1.

3. A process for the preparation of a fungicidal agent, wherein an inert additive is mixed with an N-(arylpropyl)-azolylurea of the formula I as claimed in claim 1.

4. A process for combating fungi, wherein the fungi or the materials, areas, plants or seed threatened by fungal attack are treated with a fungicidally effective amount of an N-(arylpropyl)-azolylurea of the formula I as claimed in claim 1.

5. A process for the preparation of an N-(arylpropyl)-azolylurea of the formula I as claimed in claim 1, wherein a compound of the formula

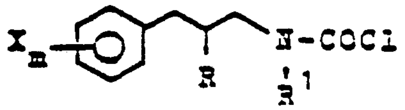 (II),

where

R, $R^1$ and X have the meanings given in claim 1, is reacted

a) with an azole of the formula

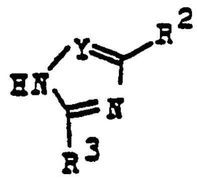 (III),

where $R^2$, $R^3$ and Y have the meanings given in claim 1, or

b) with its metal derivative of the formula

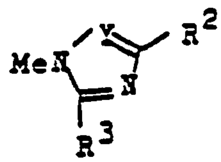 (IV),

where $R^2$, $R^3$ and Y have the meanings given in claim 1 and

Me is lithium, sodium, potassium or 1/2 calcium, or

c) with its silyl derivative of the formula

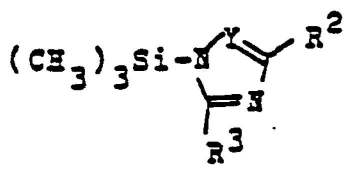 (V),

where $R^2$, $R^3$ and Y have the meanings given in claim 1.

6. A process for the preparation of an N-(arylpropyl)-azolylurea of the formula I as claimed in claim 1, wherein compound of the formula

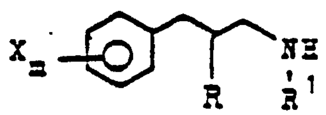 (VI),

where R, $R^1$ and X have the meanings given in claim 1, is reacted with a carbonylbisazole of the formula

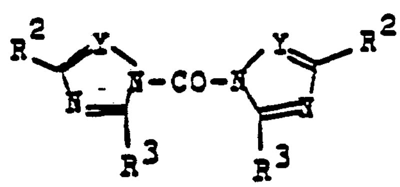 (VII),

where $R^2$, $R^3$ and Y have the meanings given in claim 1.

**Revendications** pour les Etats contractants: BE, CH, DE, FR, GB, IT, LI, NL, SE

1. N-(Arylpropyl)-azolyl-urée de la formule

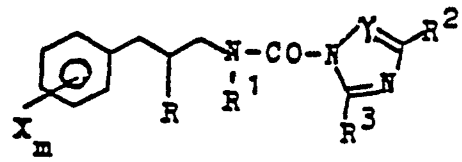 (I),

dans laquelle

R désigne un radical alkyle en $C_1$ à $C_4$,

X désigne un atome d'hydrogène ou d'halogène, un groupe nitro, cyano ou trifluorométhyle ou un radical alkyle, alcoxy ou alkylthio en $C_1$ à $C_6$ ou un groupe phényle ou phénoxy,

m est un nombre entier valant de 1 à 4, les substituants X pouvant être identiques ou différents, lorsque m est supérieur à 1,

Y représente CH ou N,

$R^1$ désigne un groupe alkyle, alcényle, alcoxyalkyle, alkylthioalkyle, cycloalkyle ou cycloalkylalkyle pouvant comporter jusqu'à 12 atomes de carbone, ou un groupe phényle ou benzyle éventuellement halo-, alkyl (en $C_1$ à $C_4$)-, alcoxy(en $C_1$ à $C_4$)-, trifluorométhyl-, nitro- ou cyano-substitué, et

$R^2$ et $R^3$, qui peuvent être identiques ou différents, désignent chacun un atome d'hydrogène ou un radical alkyle en $C_1$ à $C_5$.

2. Composition fongicide, contenant une N-(arylpropyl)-azolyl-urée de la formule I selon la revendication 1.

3. Composition fongicide, contenant une N-(arylpropyl)-azolyl-urée de la formule I selon la revendication 1 et un additif inerte.

4. Procédé de préparation d'une composition fongicide, caractérisé en ce que l'on mélange une N-(arylpropyl)-azolyl-urée de la formule I selon la revendication 1 avec un additif inerte.

5. Procédé de lutte contre des champignons, caractérisé en ce que l'on traite les champignons ou les matériaux, surfaces, plantes ou semences risquant d'être attaqués par des champignons avec une quantité efficace du point de vue fongicide d'une N-(arylpropyl)-azolyl-urée de la formule I selon la revendication 1.

6. Procédé de préparation de N-(arylpropyl)-azolyl-urées de la formule I selon la revendication 1, caractérisé en ce que l'on fait réagir un composé de la formule

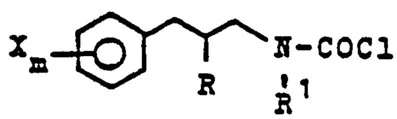 (II),

dans laquelle R, $R^1$ et X possèdent les significations définies dans la revendication 1,

a) avec un azole de la formule

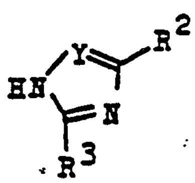 (III),

dans laquelle $R^2$, $R^3$ et Y possèdent les significations définies dans la revendication 1, ou

b) avec un dérivé de métal d'un tel azole de la formule

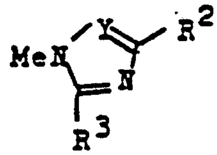 (IV),

dans laquelle $R^2$, $R^3$ et Y possèdent les significations définies dans la revendication 1 et

Me = Li, Na, K ou ½ Ca, ou

c) avec leurs dérivés silylés de la formule

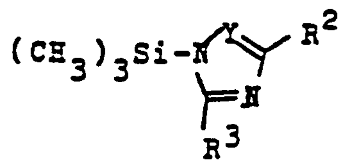 (V),

dans laquelle $R^2$, $R^3$ et Y possèdent les significations définies dans la revendication 1.

7. Procédé de préparation de N-(arylpropyl)-azolyl-urées de la formule I selon la revendication 1, caractérisé en ce que l'on fait réagir un composé de la formule

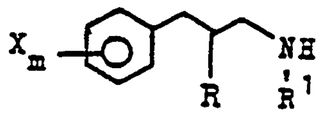 (VI),

dans laquelle R, $R^1$ et X possèdent les significations définies dans la revendication 1, avec un carbonyl-bisazole de la formule

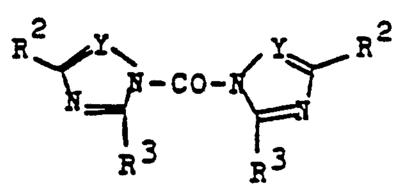 (VII),

dans laquelle $R^2$, $R^3$ et Y possèdent les significations définies dans la revendication 1.

8. N-(Arylpropyl)-azolyl-urées de la formule I selon la revendication 1, caractérisées en ce que

X est hydrogène, fluor, chlore, brome, cyano, nitro, trifluorométhyle, méthyle, éthyle, n-propyle, isopropyle, n-butyle, isobutyle, butyle secondaire, butyle tertiaire, méthoxy, éthoxy, méthyl-thio, phényle ou phénoxy,

R = méthyle ou éthyle,

$R^2$ et $R^3$ = hydrogène, méthyle, éthyle, n-propyle ou isopropyle,

Y = CH ou N,

$R^1$ est choisi parmi méthyle, éthyle, n-propyle, n-butyle, isobutyle, butyle secondaire, n-pentyle, 3-méthyl-1-butyle, 2-méthyl-1-butyle, 3-pentyle, n-hexyle, 3,3-diméthyl-1-butyle, 2,2,3-triméthyl-propyle, n-heptyle, n-octyle, 2,4,4-triméthyl-pentyle, 2-éthyl-hexyle, n-nonyle, 2,5,5-triméthyl-hexyle, 2-isopropyl-5-méthyl-hexyle, n-décyle, 3,7-diméthyl-octyle, dodécyle, cyclopropyl-méthyle, cyclopentyle, cyclohexyle, cyclohexyl-méthyle, 4-méthyl-cyclohexyle, 2-méthoxy-éthyle, 2-éthoxy-éthyle, 2-propoxy-éthyle, 2-butoxy-éthyle 3-méthoxy-propyle, 3-éthoxy-propyle, 3-propoxy-propyle, 3-butoxy-propyle, 2-méthyl-thio-éthyle, 2-éthyl-thio-éthyle, 3-méthyl-thio-propyle, allyle, 2-butène-1-yle, 2-pentène-1-yle, phényle, p-chloro-phényle, p-méthoxy-phényle, p-méthyl-phényle, benzyle, p-chloro-benzyle, 2,4-dichloro-benzyle, p-méthyl-benzyle et phényl-éthyle et

m est un nombre entier valant de 1 à 4.

**Revendications** pour l'Etat contractant: AT

1. Composition fongicide, contenant une N-(arylpropyl)-azolyl-urée de la formule

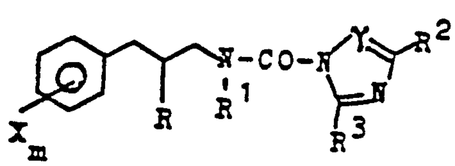 (I),

dans laquelle

R désigne un radical alkyle en $C_1$ à $C_4$,

X désigne un atome d'hydrogène ou d'halogène, un groupe nitro, cyano ou trifluorométhyle ou un radical alkyle, alcoxy ou alkylthio en $C_1$ à $C_6$ ou un groupe phényle ou phénoxy,

m est un nombre entier valant de 1 à 4, les substituants X pouvant être identiques ou différents, lorsque m est supérieur à 1,

Y représente CH ou N,

$R^1$ désigne un groupe alkyle, alcényle, alcoxyalkyle, alkylthioalkyle, cycloalkyle ou cylcoalkylalkyle pouvant comporter jusqu'à 12 atomes de carbone, ou un groupe phényle ou benzyle éventuellement halo-, alkyl(en $C_1$ à $C_4$)-, alcoxy(en $C_1$ à $C_4$)-, trifluorométhyl-, nitro- ou cyano-substitué, et

$R^2$ et $R^3$, qui peuvent être identiques ou différents, désignent chacun un atome d'hydrogène, ou un radical alkyle en $C_1$ à $C_5$.

2. Composition fongicide, contenant une N-(arylpropyl)-azolyl-urée de la formule I selon la revendication 1 et un additif inerte.

3. Procédé de préparation d'une composition fongicide, caractérisé en ce que l'on mélange une N-(arylpropyl)-azolyl-urée de la formule I selon la revendication 1 avec un additif inerte.

4. Procédé de lutte contre des champignons, caractérisé en ce que l'on traite les champignons ou les matériaux, surfaces, plantes ou semences risquant d'être attaqués par des champignons avec une quantité efficace du point de vue fongicide d'une N-(arylpropyl)-azolyl-urée de la formule I selon la revendication 1.

5. Procédé de préparation de N-(arylpropyl)-azolyl-urée de la formule I selon la revendication 1, caractérisé en ce que l'on fait réagir un composé de la formule

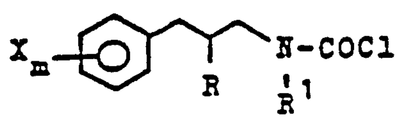 (II),

dans laquelle R, $R^1$ et X possèdent les significations définies dans la revendication 1,

a) avec un azole de la formule

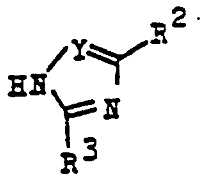 (III),

dans laquelle $R^2$, $R^3$ et Y possèdent les significations définies dans la revendication 1, ou

b) avec un dérivé de métal d'un tel azole de la formule

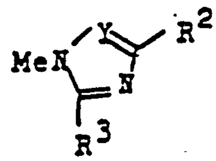 (IV),

dans laquelle $R^2$, $R^3$ et Y possèdent les significations définies dans la revendication 1 et

Me = Li, Na, K ou $\frac{1}{2}$ Ca, ou

c) avec leurs dérivés silylés de la formule

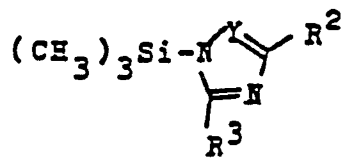 (V),

dans laquelle $R^2$, $R^3$ et Y possèdent les significations définies dans la révendication 1.

6. Procédé de préparation de N-(arylpropyl)-azolyl-urées de la formule I selon la revendication 1, caractérisé en ce que l'on fait réagir un composé de la formule

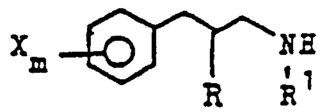

(VI),

dans laquelle R, $R^1$ et X possèdent les significations définies dans la revendication 1, avec un carbonyl-bisazole de la formule

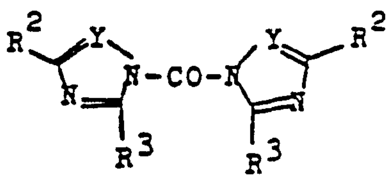

(VII),

dans laquelle $R^2$, $R^3$ et Y possèdent les significations définies dans la revendication 1.